# EUROPEAN PATENT APPLICATION

(11) **EP 3 141 247 A1**
(43) Date of publication of application: **15.03.2017**
(21) Application number: 15188232.1
(22) Date of filing: 02.10.2015
(51) Int. Cl.: A61K 9/48

(54) **MANUFACTURING METHOD FOR SOFTGEL CAPSULE CONTAINING SOLID AND LIQUID FORMULATIONS AS CORE INGREDIENT**

(30) Priority: 21.08.2015 KR 20150117682; 21.08.2015 KR 20150117683; 21.08.2015 KR 20150117684
(71) Applicant: Suheung Co., Ltd, Chungbuk 363-951 (KR)
(72) Inventor: YANG, Joo-Hwan, 461-370 Gyeonggi-do (KR); PARK, Geum Duk, 137-040 Seoul (KR)
(74) Representative: Dempster, Robert Charles

(57) **Abstract**

The present invention relates to a manufacturing method for softgel capsule containing solid and liquid formulations as core ingredient, wherein at least one pharmaceutical drug can be included in both solid formulation and liquid formulation. More particularly, this invention relates to a softgel capsule wherein liquid formulation, for example, solution and/or suspension as well as solid formulation, for example, tablet, pellet and/or sustained release dosage are present as core ingredient and its manufacturing method.

## Description

### Technical Field

The present invention relates to a manufacturing method for softgel capsule containing solid and liquid formulations as core ingredient, wherein at least one pharmaceutical drug can be included in both solid formulation and liquid formulation. More particularly, this invention relates to a softgel capsule wherein liquid formulation, for example, solution and/or suspension as well as solid formulation, for example, tablet, pellet and/or sustained release dosage are present as core ingredient and its manufacturing method.

### Description of Prior Art

Conventionally, gelatin soft capsule has been prepared by following steps of i) solubilizing the drug into liquid formulation as core ingredient; and ii) preparing and formulating the shell using gelatin. Further, the core ingredient of gelatin soft capsule can be in the form of paste, suspension as well as solubilized solution.

Further, in case of hydrophilic drug, polyethylene glycol (PEG) 200, 300, 400 or 600 or propylene glycol have been used as a solubilizer, while soybean oil including lecithin as surfactant has been used as a solubilizer in case of hydrophobic drug.

For manufacturing the sustained release formulation, the release control polymer has to be inserted in the drug formulation. The sustained release formulation can be ordinarily in the form of tablet or hard capsule, while the soft capsule cannot be available as the sustained release formulation due to its fast absorption of drug into the human body.

On the other hand, in case of tablet or hard capsule containing release control polymer, the storage stability and/or pH stability of drug tends to be declined according to the exposure of drug formulation into various external environments. Therefore, the sustained release of drug cannot be sufficiently provided if tablet or hard capsule type of sustained release formulation is administered by the lapse of 2 or 3 months after manufacturing it.

Recently, in PCT International publication WO 2012/17326 'Apparatus and process for encapsulating capsules or other solid dosage forms within capsules', the softgel capsule containing solid dosage forms selected from pellet, smaller capsule, smaller tablet, sustained release solid dosage form, immediate release solid dosage form and extended release solid dosage form has been disclosed.

Further, in this publication, an apparatus which comprises: (a) two spreader boxes; (b) two casting drums; (c) a pair of rotary dies having means for suction; (d) a liquid fill system; (e) a wedge for heating gelatine ribbons and feeding said fill; and (f) two lateral dispensing devices has been disclosed for making softgel capsules. Further, it has been disclosed that said lateral dispensing devices can include hoppers, channel guides and a grasping claw has been disclosed.

For manufacturing softgel capsules containing solid dosage forms, following steps have been disclosed, which comprises: (a) casting a gel forming composition; (b) passing said films through a pair of rotary dies having vacuum means to make pockets; (c) feeding smaller solid dosage forms into said pockets using a lateral dispensing; (d) filling said pockets with a medicine formulation in liquid form via a wedge segment; and (e) forming the capsule by sealing the pockets together.

Since the uniformed vacuum or reduced pressure have been required along the peripheral line of both rotary dies for making suitable pockets, it is not easy to produce and adjust balanced suitable pockets for feeding solid dosage forms along the peripheral line of both rotary dies. Sometimes, it will cause the inconvenience of operating said apparatus for manufacturing softgel capsules.

Further, if the applied reduced pressure is lower than required pressure, the solid dosage forms can be apart from gelatin sheet along the pockets. If the applied reduced pressure is higher than required pressure, the solid dosage forms may cling to the gelatin sheet along the pockets.

In case that the unbalanced vacuum or reduced pressure lies along the peripheral line of rotary dies, uniformed and balanced forming and sealing of softgel capsule seldom be obtained , which causes the one major defect of softgel capsules.

On the other hand, the high cost for maintaining the uniformed and balanced vacuum or reduced pressure conditions of both rotary dies can be another defect of manufacturing apparatus disclosed in WO 2012/17326.

To overcome such defects of present manufacturing method, the inventors of present invention has developed a manufacturing method and an apparatus which comprises i) only one dispensing device for inserting solid formulation; and ii) one place of pockets along the peripheral line of right side die roll, which requires only 1 vacuum or reduced pressure condition corresponding to one place of pockets. Therefore, uniformed and balanced forming and sealing of softgel capsule can be obtained in a continuous manner, which results in minimization of default of manufacturing softgel capsules.

### Problem to be solved

The problem to be solved is to develop a manufacturing method and an apparatus, which affords the segmental characteristics of apparatus structure comprising i) only one dispensing device for inserting solid formulation; and ii) one place of pockets along the peripheral line of right side die roll. Accordingly, it requires only 1 vacuum or reduced pressure condition corresponding to one place of pockets. Further, uniformed and balanced forming and sealing of softgel capsule can be fulfilled in a continuous manner.

### Means for solving the problem

The object of present invention is to provide a manufacturing method for softgel capsule containing solid and liquid formulations as core ingredient comprising the steps of: i) supplying the first gelatin sheet from the first gelatin cooling drum (10) to the upper surface of left side die roll (30) via the first transportation roller (12); ii) supplying the second gelatin sheet from second gelatin cooling drum (20) to the upper surface of left side die roll (40) via the first transportation roller (22); iii) transferring and inserting the solid formulation from solid formulation supply shooter (50) into the pocket (404) formed on reduced pressure peripheral surface (402) of right side die roll via solid formulation transferring roller (45); iv) injecting the liquid formulation at injection and sealing part (403) located at the contacting place of left side die roll and right side die roll just under the central of wedge via central liquid injection passage (801); and v) forming the softgel capsule including solid and liquid formulations inside, by heat pressing, cutting off and sealing the first and the second gelatin sheets; wherein at least one pharmaceutical drug can be included in both solid formulation and liquid formulation.

Further, the step for transferring and inserting the solid formulation comprises i) transferring the solid formulation (503) along the slope of solid formulation transferring route (503); and ii) supplying the solid formulation to the solid formulation transferring roller (45) one by one using solid formulation supply anchor (504).

Further, the step for forming pocket (404) in step iii) comprises a step for forming a vacuum part inside of right side die roll (40) to make the peripheral surface laid on reduced pressure; and a step for forming a pocket using the supplied second gelatin sheet on the reduced pressure peripheral surface (402) of right side die roll.

Further, the step for forming the softgel comprises (a) supplying the first gelatin sheet from the peripheral surface (301) of left side die roll just under the contacting surface (803) of wedge segment (80); (b) supplying the second gelatin sheet on which pocket (404) is formed from the peripheral surface (401) of right side die roll just under the contacting surface (802) of wedge segment (80), wherein the solid formulation clings to the surface of pocket; (c) injecting the liquid formulation at injection and sealing part (403) located at the contacting place of left side die roll and right side die roll just under the central of wedge via central liquid injection passage (801); and (d) heat pressing, cutting off and sealing the first and the second gelatin sheets for forming the softgel capsule including solid and liquid formulations inside.

The other object of present invention is to provide a softgel capsule manufactured by said method.

Further, said solid formulation can be any solid dosage formulation having at least one pharmaceutical drug selected from tablet, coated-tablet, pellet, microcapsule, sustained-release solid dosage or core-shell type solid dosage.

Further, said liquid formulation can be any liquid dosage formulation having at least one pharmaceutical drug selected from solution, suspension or emulsion, which can be dissolved in at least one solvent selected from water, ethanol, omega oil, PEG 200, 300, 400 or 600, propylene glycol, soybean oil or vegetable oil.

Further, said softgel capsule further contains a non-ionic surfactant or an ionic surfactant to increase the compatibility between solid formulation and liquid formulation.

### Advantageous effect

The outstanding advantageous effect of the present invention is to provide a manufacturing method and an apparatus, which affords the segmental characteristics of apparatus structure comprising i) only one dispensing device for inserting solid formulation; and ii) one place of pockets along the peripheral line of right side die roll. Accordingly, it requires only 1 vacuum or reduced pressure condition corresponding to one place of pockets. Further, the manufacturing apparatus of present application can also provide a uniformed and balanced forming and sealing of softgel capsule in a continuous manner.

On the other hand, the present invention is to provide a softgel capsule containing solid and liquid formulations as core ingredient, wherein at least one pharmaceutical drug can be included in both solid formulation and liquid formulation.

### Brief description of drawings

FIG. 1 shows a schematic overall view of the apparatus for manufacturing softgel capsule containing solid and liquid formulations of the present invention.
FIG. 2 shows a detailed view of the softgel forming part of manufacturing apparatus of the present invention.
FIG. 3 shows a plan view of solid formulation supply shooter of the present invention.
FIG. 4a shows a side view of solid formulation supply shooter of the present invention.
FIG. 4b shows a schematic view indicating the operation for of solid formulation transferring roller of the present invention.
FIG. 5 shows a detailed view of solid formulation supply shooter of present invention.
FIG. 6a shows a schematic view of softgel capsules arranged just after manufacturing according to the method of the present invention.
FIG. 6b shows a schematic view of softgel capsule containing solid and liquid formulations of the present invention.

### Preferred embodiment of invention

The present invention relates a manufacturing method for softgel capsule containing solid and liquid formulations as core ingredient comprising the steps of: i) supplying the first gelatin sheet from the first gelatin cooling drum (10) to the upper surface of left side die roll (30) via the first transportation roller (12); ii) supplying the second gelatin sheet from second gelatin cooling drum (20) to the upper surface of left side die roll (40) via the first transportation roller (22); iii) transferring and inserting the solid formulation from solid formulation supply shooter (50) into the pocket (404) formed on reduced pressure peripheral surface (402) of right side die roll via solid formulation transferring roller (45); iv) injecting the liquid formulation at injection and sealing part (403) located at the contacting place of left side die roll and right side die roll just under the central of wedge via central liquid injection passage (801); and v) forming the softgel capsule including solid and liquid formulations inside, by heat pressing, cutting off and sealing the first and the second gelatin sheets; wherein at least one pharmaceutical drug can be included in both solid formulation and liquid formulation.

On the other hand, the present invention also relates to a softgel capsule containing solid and liquid formulations as core ingredient manufactured by above described method.

Further, said solid formulation having at least one pharmaceutical drug can be any solid dosage formulation selected from tablet, coated-tablet, pellet, microcapsule, sustained-release solid dosage or core-shell type solid dosage. Further, said liquid formulation having at least one pharmaceutical drug can be any liquid dosage formulation selected from solution, suspension or emulsion, which can be dissolved in at least one solvent selected from water, ethanol, omega oil, PEG 200, 300, 400 or 600, propylene glycol, soybean oil or vegetable oil.

Further, said softgel capsule further contains a non-ionic surfactant or an ionic surfactant to increase the compatibility between solid formulation and liquid formulation. Non-ionic surfactant may be preferred. Tween or lecithin can be generally used.

On the other hand, solid formulation and liquid formulation can include the generic drug or ethical drug as effective ingredient. A generic drug can be non-steroidal antiinflammatory drug or anti-histamine drug, while an ethical drug can be anti-hypertension, anti-hyper cholesterol, anti-hyper triglyceride and/or anti-diabetes. If solid formulation is a sustained release drug dosage, the polymer for controlling the release can be included.

On the other hand, said liquid formulation having at least one pharmaceutical drug can be dissolved in at least one solvent selected from water, ethanol, PEG 200, 300, 400 or 600, propylene glycol, if the drug in liquid formulation is water-soluble or hydrophilic. However, said liquid formulation can be dissolved in at least one solvent selected from omega oil, soybean oil or vegetable oil, if the drug in liquid formulation is lipid-soluble or hydrophobic. Further, polyvinyl pyrrolidone can be included as binder in liquid formulation.

The shell component of softgel capsule has been generally made by gelatin. Of course, glycerin and/or sorbitol can be included in gelatin shell component as plasticizer.

The present invention can be explained more specifically by the reference of drawings.

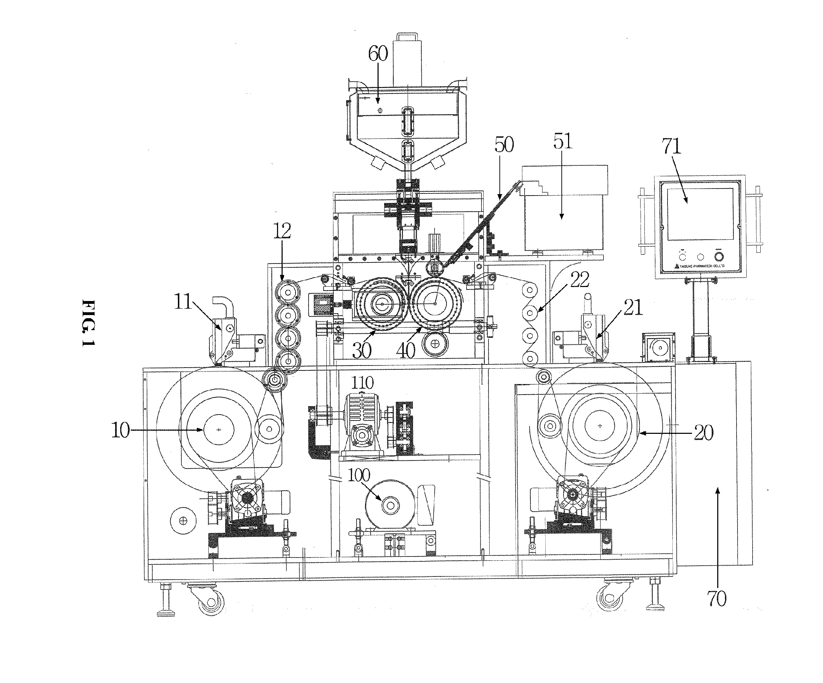

FIG. 1 shows a schematic overall view of the apparatus for manufacturing softgel capsule containing solid and liquid formulations of the present invention. As shown in FIG. 1, the apparatus for manufacturing softgel capsule of the present invention has been composed of 4 part units. The structure of 4 part units can be explained as follows.

The first part unit is a first gelatin sheet supplying unit for supplying the first gelatin sheet from the first gelatin cooling drum (10) to the upper surface of left side die roll (30) via the first transportation roller (12).

The second part unit is a second gelatin sheet supplying unit for supplying the second gelatin sheet from second gelatin cooling drum (20) to the upper surface of left side die roll (40) via the first transportation roller (22).

The third part unit is a solid formulation transferring and inserting unit for transferring and inserting the solid formulation from solid formulation supply shooter (50) into the pocket (404) formed on reduced pressure peripheral surface (402) of right side die roll via solid formulation transferring roller (45). Further, the solid formulation can be any solid dosage formulation, which can be at least one formulation selected from tablet, coated-tablet, pellet, microcapsule, sustained-release solid dosage or core-shell type solid dosage.

The fourth part unit is a softgel capsule forming unit for forming the softgel capsule including solid and liquid formulations inside, by heat pressing, cutting off and sealing the first and the second gelatin sheets. In this unit, the injection of the liquid formulation also has been made at injection and sealing part (403) located at the contacting place of left side die roll and right side die roll just under the central of wedge via central liquid injection passage (801).

The characteristics of the present apparatus for manufacturing softgel capsule comprise i) only one dispensing device for inserting solid formulation; and ii) one place of pockets along the peripheral line of right side die roll. In other words, the present apparatus for manufacturing softgel capsule requires only 1 vacuum or reduced pressure condition corresponding to one place of pockets.

As a result, the shape of softgel capsule manufactured by the present manufacturing apparatus can be uniformed and balanced. Further, both uniformed weight of solid formulation and uniformed volume of liquid formulation can be achieved and afforded.

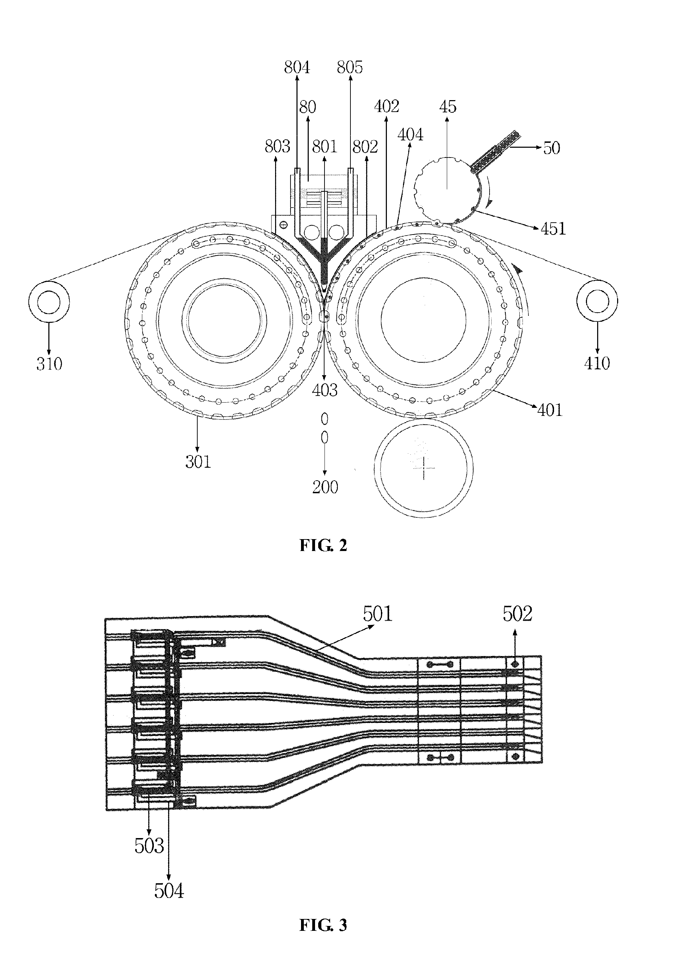

FIG. 2 shows a detailed view of the softgel forming part of manufacturing apparatus of the present invention.

In FIG. 2, all segments for forming the softgel have been illustrated. Further, following forming steps can be also interpreted. In detail, forming steps can comprise
(a) supplying the first gelatin sheet from the peripheral surface (301) of left side die roll just under the contacting surface (803) of wedge segment (80);
(b) supplying the second gelatin sheet on which pocket (404) is formed from the peripheral surface (401) of right side die roll just under the contacting surface (802) of wedge segment (80), wherein the solid formulation clings to the surface of pocket;
(c) injecting the liquid formulation at injection and sealing part (403) located at the contacting place of left side die roll and right side die roll just under the central of wedge via central liquid injection passage (801); and
(d) heat pressing, cutting off and sealing the first and the second gelatin sheets for forming the softgel capsule including solid and liquid formulations inside.

Further, the pocket can be prepared by the steps comprising i) forming a vacuum part inside of right side die roll (40) to make the peripheral surface laid on reduced pressure; and ii) forming a pocket using the supplied second gelatin sheet on the reduced pressure peripheral surface (402) of right side die roll.

Further, for supplying the solid formulation into the pocket, following steps can be required i) transferring the solid formulation (503) along the slope of solid formulation transferring route (503); and ii) supplying the solid formulation to the solid formulation transferring roller (45) one by one using solid formulation supply anchor (504).

On the other hand, the present manufacturing method of softgel capsule has overcome the conventional problem caused by the unbalanced vacuum or reduced pressure lying along the peripheral surface of rotary dies, by introducing the segmental improvements of apparatus structure containing i) only one dispensing device for inserting solid formulation; and ii) one place of pockets along the peripheral line of right side die roll. Therefore, only 1 vacuum or reduced pressure condition has been required in right side die roll corresponding to one place of pockets.

Therefore, the softgel capsule manufacturing method by the present manufacturing apparatus can reduce the cost for manufacturing softgel capsule, because the consumption of electricity has been declined by saving the energy resulted from only 1 vacuum or reduced pressure condition equipped in right side die roll.

Further, the softgel capsule without any defect regarding the shape and quality can be achieved by introducing manufacturing method of the present application.

FIG. 3 shows a plan view of solid formulation supply shooter of the present invention.

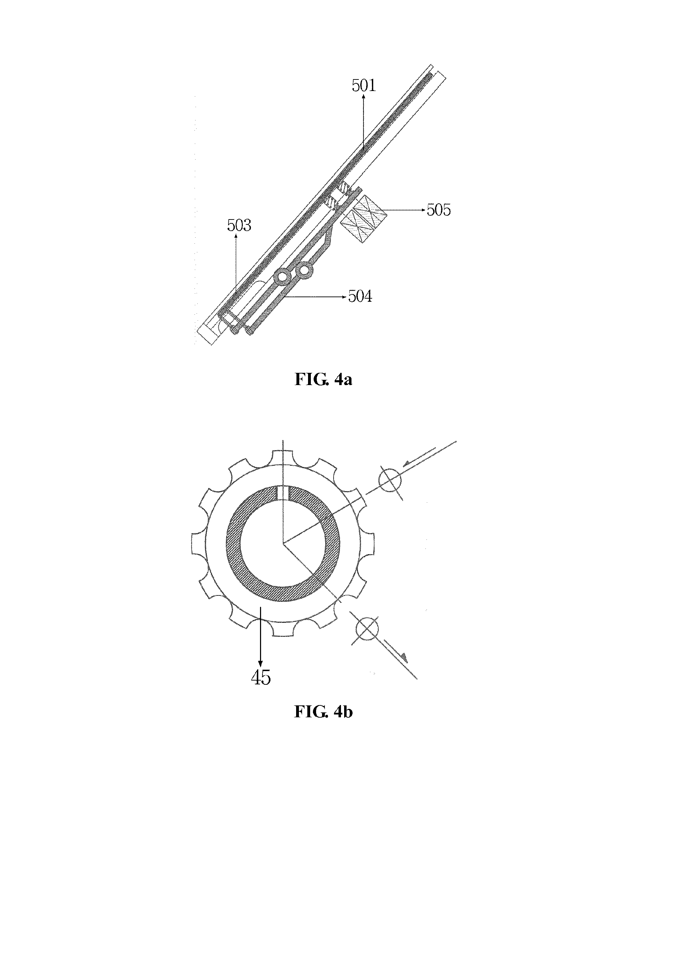

FIG. 4a shows a side view of solid formulation supply shooter of the present invention.

As shown in these figures, the transfer of solid formulation can be made with following steps comprising i) transferring the solid formulation (503) along the slope of solid formulation transferring route (503); and ii) supplying the solid formulation to the solid formulation transferring roller (45) one by one using solid formulation supply anchor (504).

FIG. 4b shows a schematic view indicating the operation for of solid formulation transferring roller of the present invention.

The solid formulation supplied from solid formulation supply shooter (50) has been transferred into peripheral surface (402) of right side die roll via solid formulation transferring roller (45). As shown in FIG. 4b, solid formulation has been supplied into peripheral surface of circulating solid formulation transferring roller (45) and it has been inserted into the groove of solid formulation transferring roller (45). Finally, the solid formulation has been released into peripheral surface (402) of right side die roll.

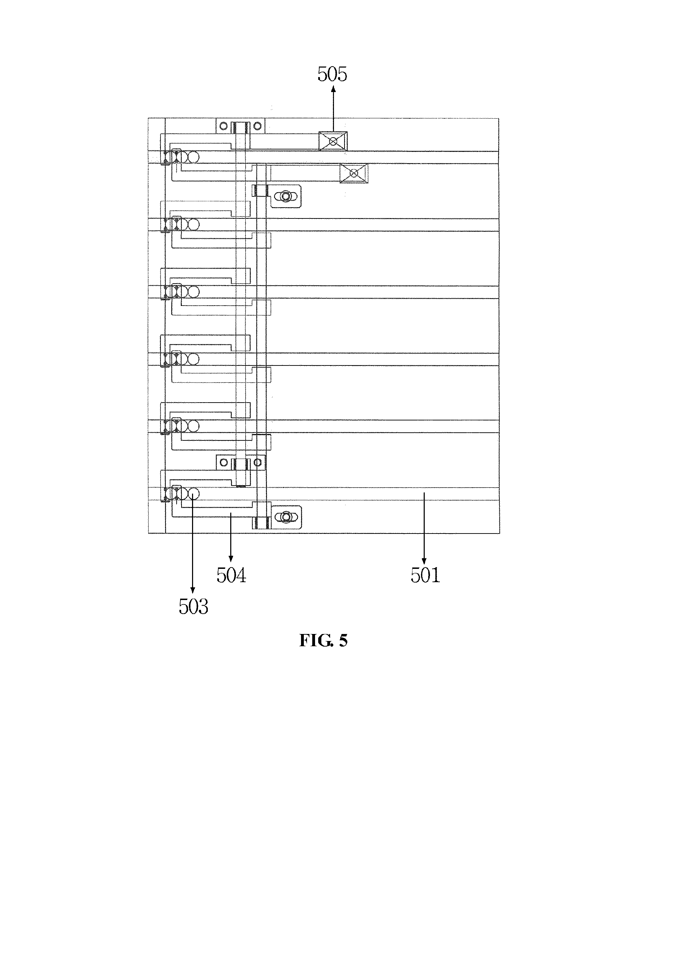

FIG. 5 shows a detailed view of solid formulation supply shooter of present invention.

As shown in FIG. 5, the solid formulation has been transferred into solid formulation transferring roller (45) one by one using solid formulation supply anchor (504) according to the slope of solid formulation supply shooter (50).

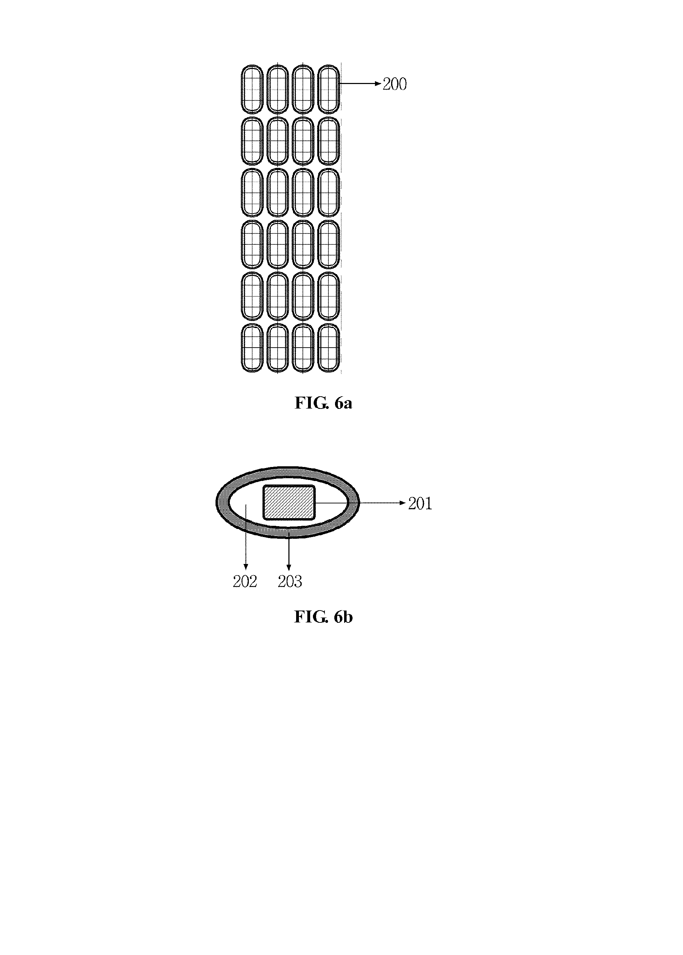

FIG. 6a shows a schematic view of softgel capsules arranged just after manufacturing according to the method of the present invention.

As shown in FIG. 6a, the softgel capsules just after completion of manufacturing from the softgel manufacturing apparatus of the present invention have been arranged up and down in a symmetric manner as well as right and left in a symmetric manner. This arranged shape of softgel capsules is different from conventional softgel capsule, because the arranged safe of conventional softgel capsule has zigzag shape without symmetric manner.

On the other hand, the symmetrically arranged shape of softgel capsules can prevent the softgel capsule to be attached or pressed, which causes the change of uniformed shape.

FIG. 6b shows a schematic view of softgel capsule containing solid and liquid formulations of the present invention.

As shown in FIG. 6b, the softgel capsule can contain both solid formulation (201) and liquid formulation (202) as core ingredient. The shell (203) has been made by gelatin. Further, the liquid formulation can be in the form of solution, suspension or emulsion. Of course, at least one pharmaceutical drug can be included in both solid formulation and liquid formulation.

**Reference Numerals**

| | | | |
|---|---|---|---|
| 10: | The first gelatin cooling drum | 11: | The first gelatin spreader box |
| 12: | The first transportation roller | 20: | The second gelatin cooling drum |
| 21: | The second gelatin spreader box | 22: | The second transportation roller |
| 30: | Left side die roll | 40: | Right side die roll |
| 45: | Solid formulation transferring roller | 50: | Solid formulation supply shooter |
| 51: | Solid formulation supply hopper | 60: | Liquid formulation heating hopper |
| 70: | System controller | 71: | Display panel |
| 80: | Wedge segment | 100: | Main motor |
| 110: | Main speed reducer | 200: | Arranged softgel capsule |
| 201: | Solid formulation | 202: | Liquid formulation |
| 203: | Gelatin shell | 301: | Peripheral surface of left side die roll |
| 310: | Guide roller | 401: | Peripheral surface of right side die roll |
| 402: | Reduced pressure peripheral surface | 403: | Injection and sealing part |
| 404: | Pocket | 410: | Guide roller |
| 451: | Transferred solid formulation | 501: | Solid formulation transferring route |
| 502: | Connecting part of solid formulation supply hopper | | |
| 503: | Inserted solid formulation | 504: | Supply anchor for solid formulation |
| 505: | Solid formulation supply shooter support | | |
| 801: | Central part liquid injection passage | 802: | Contacting face of wedge segment |
| 803: | Contacting face of wedge segment | 804: | Side part liquid injection passage |
| 805: | Side part liquid injection passage | | |

## Claims

1. A manufacturing method for softgel capsule containing solid and liquid formulations as core ingredient comprising the steps of:
i) supplying the first gelatin sheet from the first gelatin cooling drum (10) to the upper surface of left side die roll (30) via the first transportation roller (12);
ii) supplying the second gelatin sheet from second gelatin cooling drum (20) to the upper surface of left side die roll (40) via the first transportation roller (22);
iii) transferring and inserting the solid formulation from solid formulation supply shooter (50) into the pocket (404) formed on reduced pressure peripheral surface (402) of right side die roll via solid formulation transferring roller (45);
iv) injecting the liquid formulation at injection and sealing part (403) located at the contacting place of left side die roll and right side die roll just under the central of wedge via central liquid injection passage (801); and
v) forming the softgel capsule including solid and liquid formulations inside, by heat pressing, cutting off and sealing the first and the second gelatin sheets;
wherein at least one pharmaceutical drug can be included in both solid formulation and liquid formulation.

2. The manufacturing method for softgel capsule according claim 1, wherein the step for transferring and inserting the solid formulation comprises i) transferring the solid formulation (503) along the slope of solid formulation transferring route (503); and ii) supplying the solid formulation to the solid formulation transferring roller (45) one by one using solid formulation supply anchor (504).

3. The manufacturing method for softgel capsule according claim 1, wherein the step for forming pocket (404) in step iii) comprises a step for forming a vacuum part inside of right side die roll (40) to make the peripheral surface laid on reduced pressure; and a step for forming a pocket using the supplied second gelatin sheet on the reduced pressure peripheral surface (402) of right side die roll.

4. The manufacturing method for softgel capsule according claim 1, wherein the step for forming the softgel comprises
(a) supplying the first gelatin sheet from the peripheral surface (301) of left side die roll just under the contacting surface (803) of wedge segment (80);
(b) supplying the second gelatin sheet on which pocket (404) is formed from the peripheral surface (401) of right side die roll just under the contacting surface (802) of wedge segment (80), wherein the solid formulation clings to the surface of pocket;
(c) injecting the liquid formulation at injection and sealing part (403) located at the contacting place of left side die roll and right side die roll just under the central of wedge via central liquid injection passage (801); and
(d) heat pressing, cutting off and sealing the first and the second gelatin sheets for forming the softgel capsule including solid and liquid formulations inside.

5. The softgel capsule manufactured according to the method of claim 1.

6. The softgel capsule according to claim 5, wherein said solid formulation having at least one pharmaceutical drug can be any solid dosage formulation selected from tablet, coated-tablet, pellet, microcapsule, sustained-release solid dosage or core-shell type solid dosage.

7. The softgel capsule according to claim 5, wherein said liquid formulation having at least one pharmaceutical drug can be any liquid dosage formulation selected from solution, suspension or emulsion, which can be dissolved in at least one solvent selected from water, ethanol, omega oil, PEG 200, 300, 400 or 600, propylene glycol, soybean oil or vegetable oil.

8. The softgel capsule according to claim 6, wherein said softgel capsule further contains a non-ionic surfactant or an ionic surfactant to increase the compatibility between solid formulation and liquid formulation.
